(19) Europäisches Patentamt
European Patent Office
Office européen des brevets

(11) EP 0 767 780 B1

(12) EUROPÄISCHE PATENTSCHRIFT

(45) Veröffentlichungstag und Bekanntmachung des Hinweises auf die Patenterteilung:
**15.12.1999 Patentblatt 1999/50**

(21) Anmeldenummer: **95923331.3**

(22) Anmeldetag: **16.06.1995**

(51) Int Cl.[6]: **C07C 237/06**, C07C 237/08, C07D 311/58, C07C 269/04, A01N 37/18, A01N 43/16, C07C 271/22

(86) Internationale Anmeldenummer:
**PCT/EP95/02321**

(87) Internationale Veröffentlichungsnummer:
**WO 96/00718 (11.01.1996 Gazette 1996/03)**

# (54) AMINOSÄURE-DERIVATE UND IHRE VERWENDUNG ALS SCHÄDLINGSBEKÄMPFUNGSMITTEL

AMINO ACID DERIVATIVES AND THEIR USE AS PESTICIDES

DERIVES D'ACIDE AMINE ET LEUR UTILISATION COMME PESTICIDES

(84) Benannte Vertragsstaaten:
**BE CH DE ES FR GB GR IE IT LI NL PT**

(30) Priorität: **28.06.1994 DE 4422567**
**17.01.1995 DE 19501175**

(43) Veröffentlichungstag der Anmeldung:
**16.04.1997 Patentblatt 1997/16**

(60) Teilanmeldung: **98122727.5 / 0 911 318**

(73) Patentinhaber: **BAYER AG**
**51368 Leverkusen (DE)**

(72) Erfinder:
- **STELZER, Uwe**
  **D-51381 Leverkusen (DE)**
- **CASSER, Carl**
  **D-51061 Köln (DE)**
- **SEITZ, Thomas**
  **D-40764 Langenfeld (DE)**

(56) Entgegenhaltungen:

| | |
|---|---|
| **EP-A- 0 274 453** | **EP-A- 0 398 072** |
| **EP-A- 0 550 788** | **EP-A- 0 587 110** |
| **WO-A-93/01166** | **US-A- 3 676 492** |

- **JOURNAL OF CHROMATOGRAPHY, Bd.395, 12. Juni 1987, AMSTERDAM, NL Seiten 597 - 608 C. GAGET ET. AL. 'Separation of the enantiomers of substituted putrescine and cadaverine analogues by gas chromatography on chiral and achiral stationary phases.'**
- **JOURNAL OF CHROMATOGRAPHY, Bd.395, 12. Juni 1987, AMSTERDAM, NL Seiten 569 - 90 H. BRÜCKNER ET. AL. 'Determination of alpha-alkyl-alpha amino acids and alpha amino alcohols by chiral phase capillary gas chromatography and reversed phase high performance liquid chromatography.'**
- **JOURNAL OF CHROMATOGRAPHY, Bd.555, 30. August 1991, AMSTERDAM, NL Seiten 81 - 95 H BRÜCKNER ET. AL. 'High-performance liquid chromatographic separation of DL-amino acids derivatized with chiral variants of Sanger's reagent.'**
- **JOURNAL OF HIGH RESOLUTION CHROMATOGRAPHY AND CHROMATOGRAPHY COMMUNICATIONS, Bd.11, Nr.11, November 1988, HEIDELBERG DE Seiten 787 - 92 H. FRANK 'Chiral stationary phase for capillary gas chromatography: towards higher selectivity and stability'**
- **CHEMICAL ABSTRACTS, vol. 67, no. 25, 18. Dezember 1967, Columbus, Ohio, US; abstract no. 117279x, SHCHUKINA, L.A. ET. AL. 'Amino acid and peptide derivatives of indoles. II. Synthesis and properties of 5-methoxytryptamine analogs.' Seite 11067 ;Spalte 1 ; & ZH. OBSHCH. KHIM.,, Bd.37, Nr.3 Seiten 578 - 82**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **CHEMICAL ABSTRACTS, vol. 83, no. 2, 14. Juli 1975, Columbus, Ohio, US; abstract no. 10920c, KISELVA, I.D. ET. AL. 'Amino acid derivatives of phenylalkylamines. 3. Acylation of beta-phenylethylamines by alpha-amino acids.' Seite 2 ;Spalte 1 ; & IZV. AKAD. NAUK. SSSR, SER. KHIM., Nr.2, 1975 Seiten 424 - 7**

Bemerkungen:

Die Akte enthält technische Angaben, die nach dem Eingang der Anmeldung eingereicht wurden und die nicht in dieser Patentschrift enthalten sind.

**Beschreibung**

Die Erfindung betrifft neue Aminosäure-Derivate, ein Verfahren zu ihrer Herstellung und ihre Verwendung als Schädlingsbekämpfungsmittel, insbesondere als Fungizide sowie als Zwischenprodukte zur Herstellung von bekannten, fungizid wirksamen substituierten Aminosäure-Derivaten.
Es ist bereits bekannt, daß bestimmte substituierte Aminosäure-Derivate fungizide Eigenschaften aufweisen und erhalten werden, indem man substituierte Aminosäuren mit entsprechenden Aminen umsetzt (vgl. EP-A 0 472 995).
Des weiteren sind bestimmte Amine von substituierten Aminosäuren bekannt (vgl. EP-A 0 587 110 und EP-A-0,550,788).
Es wurden nun neue Verbindungen der allgemeinen Formel

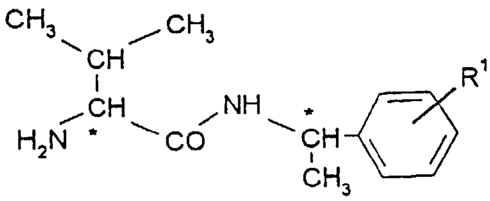

(I)

in welcher

R[1] für Chlor, Methyl, Ethyl oder Methoxy steht,

bzw. deren Salze,
gefunden
Aryl steht im folgenden vorzugsweise für jeweils unsubstituiertes oder substituiertes Phenyl, Benzothiophen oder Benzofuran, wobei als Substituenten vorzugsweise Halogen, Cyano, Alkyl, Halogenalkyl, Alkoxy und Halogenalkoxy in Frage kommen.
Alkyl steht im folgenden vorzugsweise für geradkettiges oder verzweigtes Alkyl mit 1 bis 6, insbesondere 1 bis 4 Kohlenstoffatomen wie Methyl, Ethyl, n-Propyl, n-, i-, t-Butyl und insbesondere i-Propyl und s-Butyl sowie für cyclisches Alkyl mit 3 bis 6 Kohlenstoffatomen, wie inbesondere Cyclopentyl.
Alkylen steht im folgenden vorzugsweise für geradkettiges oder verzweigtes Alkylen mit 1 bis 6, vorzugsweise 1-4 Kohlenstoffatome wie insbesondere 1,1-Ethylen, 1,2-Ethylen, 1,1-Propylen, 1-Methyl-1,2-ethylen, 2-Methyl-1,2-ethylen, 1,1-Dimethyl-methylen.
Die Verbindungen der Formel (I) enthalten zwei Chiralitätszentren und können somit in verschiedenen Enantiomeren- und Diastereomerengemischen vorliegen, die gegebenenfalls in üblicher Art und Weise getrennt werden können. Sowohl die reinen Enantiomeren und Diastereomeren, als auch die Gemische werden erfindungsgemäß beansprucht.
Im folgenden wird der Einfachheit halber stets von Verbindungen der Formel (I) gesprochen, obwohl sowohl die reinen Verbindungen, als auch die Gemische mit unterschiedlichen Anteilen an isomeren, enantiomeren und diastereomeren Verbindungen gemeint sind.
Entsprechendes gilt für die noch folgenden Verbindungen der Formeln (m) und (IV).
Weiterhin wurde gefunden, daß man die Verbindungen der Formel (I)

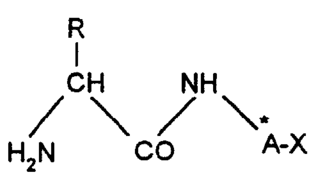

(I)

in welcher

A für geradkettiges oder verzweigtes Alkylen steht

X für unsubstituiertes oder substituiertes Aryl oder für unsubstituiertes oder substituiertes Heterocyclyl steht, und

R für geradkettiges, verzweigtes oder cyclisches Alkyl steht,
bzw. deren Salze, in überraschend guter Ausbeute erhält, wenn man Oxazolidindione der Formel (II)

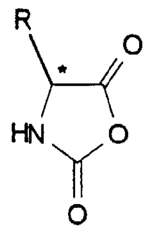

( II )

in welcher

R die oben angegebene Bedeutung hat,

mit Aminen der allgemeinen Formel (III)

$$H_2N\text{-}A\text{-}X \quad (III)$$

in welcher

A und X die oben angegebenen Bedeutungen haben,

in Gegenwart eines Verdünnungsmittels und gegebenenfalls in Gegenwart eines Reaktionshilfsmittels umsetzt.

Außerdem wurde gefunden, daß die neuen Aminosäure-Derivate der Formel (I) als Schädlingsbekämpfungsmittel verwendet werden können.

Bevorzugt sind diejenigen Verbindungen der Formel (I), in welchen es sich bei der zugrundeliegenden Aminosäure um die Verbindung mit der L-Konfiguration handelt, und das eingesetzte Amin der Formel (III), entweder racemisch ist oder die R-Konfiguration oder die S-Konfiguration am asymmetrischen Kohlenstoffatom aufweist.

Die zur Durchführung des erfindungsgemäßen Verfahrens als Ausgangsstoff zu verwendenden Oxazolidindione der Formel (II) sind bekannt (vgl. Angew. Chem. 93, 793 (1981)) und können nach dem dort beschriebenen Verfahren erhalten werden, indem man die entsprechenden Aminosäuren in üblicher Art und Weise mit Phosgen umsetzt.

Die außerdem für die Durchführung des erfindungsgemäßen Verfahrens als Ausgangsstoffe zu verwendenden Amine sind durch die Formel (III) allgemein definiert.

Die Amine der Formel (III) sind allgemein bekannte Verbindungen der organischen Chemie.

Als Verdünnungsmittel kommen für das erfindungsgemäße Verfahren inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Das erfindungsgemäße Verfahren wird gegebenenfalls in Gegenwart eines Puffers als Reaktionshilfsmittel durchgeführt. Hierbei können alle üblichen Puffersysteme eingesetzt werden, wie insbesondere Natriumboranat/Borsäure.

Die Temperaturen können bei der Durchführung des erfindungsgemäßen Verfahrens in einem größeren Bereich variiert werden. Im allgemeinen arbeitet man bei Temperaturen von -100 bis +150°C, vorzugsweise von -60 bis +100°C.

Bei der Durchführung des erfindungsgemäßen Verfahrens setzt man je Mol Oxazolidinon der Formel (II) im allgemeinen 1 bis 10 Mol, vorzugsweise 1 bis 5 Mol an Amin der Formel (III) ein.

In einer besonderen Durchführungsform des erfindungsgemäßen Verfahrens wird das Amin der Formel (III) gleichzeitig auch als Verdünnungsmittel eingesetzt.

Die Aufarbeitung und Isolierung der Verbindungen erfolgt nach allgemein üblichen und bekannten Methoden.

Die Erfindung umfaßt sowohl die reinen Isomeren als auch die Gemische der Verbindungen der Formel (I). Diese Gemische können nach gebräuchlichen Methoden, z.B. selektive Kristallisation aus geeigneten Lösungsmitteln oder Chromatographie an Kieselgel oder Aluminiumoxid in die Komponenten aufgetrennt werden. Racemate können

nach üblichen Methoden in die einzelnen Enantiomeren aufgetrennt werden, so z.B. durch Salzbildung mit optisch aktiven Säuren wie Camphersulfonsäure oder Dibenzoylweinsäure und selektive Kristallisation oder durch Derivatisierung mit geeigneten, optisch aktiven Reagenzien, Trennung der diastereomeren Derivate und Rückspaltung oder Trennung an optisch aktivem Säulenmaterial.

Die erfindungsgemäßen Verbindungen der Formel (I) weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

In entsprechenden Aufwandmengen zeigen die erfindungsgemäßen Verbindungen auch eine herbizide bzw. insektizide Wirkung.

Beispielhaft aber nicht begrenzend seien einige Erreger von pilzlichen Krankheiten, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens. Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent, vorzugsweise zwischen 0,5 und 90 %, Wirkstoff.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Für die Mischungen kommen beispielsweise infrage:

**Fungizide:**

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazole-5-carboxanilid; 2,6-Dichloro-N-(4-trifluoromethylbenzyl)benzamid; (E)-2-Methoxyimino-N-methyl-2-{2-phenoxyphenyl)-acetamid; 8-Hydroxyquinolinsulfat; Methyl-(E)-2-(2-[6-(2-cyanophenoxy)pyrimidin-4-yloxy]phenyl}-3-methoxyacrylat; Methyl-(E)-methoximino[alpha-(o-tolyloxy)-o-tolyl] acetat; 2-Phenylphenol (OPP), Aldimorph, Ampropylfos, Anilazin, Azaconazol,

Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Didobutrazol, Diclofluanid, Didomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickeldimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

**Bakterizide:**

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.
Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.
Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.
Bei der Saatgutbehandlung werden im allgemeinen Wirkstoffmengen von 0,001 bis 50 g je Kilogramm Saatgut, vorzugsweise 0,01 bis 10 g benötigt.
Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.
Die erfindungsgemäßen Verbindungen der Formel (I) eignen sich insbesondere auch als Zwischenprodukte

zur Herstellung von bekannten, fungizid wirksamen substituierten Aminosäuren-Derivaten der allgemeinen Formel (IV)

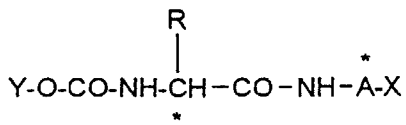

(IV)

in welcher
R, A und X die oben angegebenen Bedeutungen haben und

Y für Aryl, Aralkyl oder Alkyl, vorzugsweise i-Propyl und s-Butyl steht oder für jeweils gegebenenfalls durch Chlor, Methyl und/oder Methoxy, substituiertes Phenyl, Benzyl oder Cyclopentyl steht.

Die Aminosäure-Derivate der Formel (IV) werden dabei erhalten, wenn man die Verbindungen der Formel (I)

a) mit Säure-Derivaten der allgemeinen Formel (V)

Y-O-CO-Z (V)

in welcher

Y die oben angegebene Bedeutung hat und

Z für Halogen oder die Gruppierung -O-CO-OY steht,
in Gegenwart eines Verdünnungsmittels sowie gegebenenfalls in Gegenwart eines Säurebindemittels und gegebenenfalls in Gegenwart eines Katalysators bei Temperaturen zwischen -100 und +120°C umsetzt;
oder

b) zunächst mit Phosgen in Gegenwart eines Verdünnungsmittels und gegebenenfalls in Gegenwart eines Säurebindemittels bei Temperaturen zwischen -100 und +120°C umsetzt und anschließend, ohne Isolierung der intermediär gebildeten Isocyanate bzw. Carbamoylchloride, mit Alkoholen der allgemeinen Formel (VI)

Y-OH (VI)

in welcher

Y die oben angegebene Bedeutung hat,

bei Temperaturen zwischen -100 und +120°C umsetzt.

Die Verfahrensvarianten (a) und (b) liefern dabei die Verbindungen der Formel (IV) in nahezu quantitativer Ausbeute.

Ein besondere Vorteil liegt desweiteren in der Gesamtheit der Verfahren, d.h. in der direkten Kombination des erfindungsgemäßen Verfahrens zur Herstellung der Verbindungen der Formel (I) und deren weiteren Umsetzung gemäß den Verfahrensvarianten (a) und (b).

Dabei ist es auch möglich, die Verbindungen der Formel (I) ohne Zwischenisolierung direkt weiter umzusetzen.

Die für die Durchführung der erfindungsgemäßen Verfahrensvariante (a) als Ausgangsstoffe zu verwendenden Säure-Derivate sind durch die Formel (V) allgemein definiert. In dieser Formel hat Y die oben genannten Bedeutungen. Z steht vorzugsweise für Chlor, Brom oder die Gruppierung -O-CO-OY.

Die Säure-Derivate der Formel (V) sind allgemein bekannte Verbindungen der organischen Chemie.

Die für die Durchführung der erfindungsgemäßen Verfahrensvariante (b) als Ausgangsstoffe zu verwendenden Alkohole sind durch die Formel (VI) allgemein definiert. In dieser Formel hat Y die oben genannten Bedeutungen.

Die Alkohole der Formel (VI) sind allgemein bekannte Verbindungen der organischen Chemie.

Als Verdünnungsmittel kommen für die erfindungsgemäßen Verfahrensvarianten (a) und (b) inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Als Säurebindemittel kommen für die erfindungsgemäßen Verfahrensvarianten (a) und (b) übliche anorganische und organische Säurebinder in Frage. Hierzu gehören vorzugsweise tertiäre Amine, wie Triethylamin, Pyridine oder N-Methylpiperidin, sowie anorganische Basen, z.B. Metallhydroxide wie Natrium- und Kaliumhydroxid oder Metallcarbonate wie Natriumcarbonat oder Calciumcarbonat.

Die erfindungsgemäße Verfahrensvariante (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung der Verfahrensvarianten (a) und (b) in einem größeren Bereich variiert werden. Im allgemeinen arbeitet man bei Temperaturen von -100 bis +120°C, vorzugsweise von -60 bis +50°C.

Bei der Durchführung der erfindungsgemäßen Verfahrensvariante (a) setzt man je Mol der Verbindung der Formel (I) im allgemeinen 1 bis 2 Mol, vorzugsweise 1 bis 1,5 Mol an Säure-Derivat der Formel (V) ein.

Bei der Durchführung der erfindungsgemäßen Verfahrensvariante (b) arbeitet man vorzugsweise in äquimolaren Mengen. In manchen Fällen erweist es sich als vorteilhaft, anstelle von Phosgen mit Di- oder Triphosgen zu arbeiten bzw. einen Überschuß an Alkohol der Formel (VI) einzusetzen.

Die nach den Verfahrensvarianten (a) und (b) erhaltenen substituierten Aminosäure-Derivate der allgemeinen Formel (IV) können nach gebräuchlichen Methoden, wie z.B. Kristallisation aus einem geeigneten Lösungsmittel oder Chromatographie an Kieselgel bzw. Aluminiumoxid aufgereinigt und in die Komponenten aufgetrennt werden. Racemate können nach üblichen Methoden in die einzelnen Enantiomeren aufgetrennt werden.

**Herstellungsbeispiele**

**Beispiel 1**

L-Valin-4-methylpheneth-1-yl-amid

23,6 g (0,175 Mol) 4-Methylpheneth-1-yl-amin in 100 ml Acetonitril werden bei -10°C innerhalb von 4 bis 6 Stunden mit 5,0 g (0,035 Mol) 4-Isopropyl-2,5-oxazolidindion versetzt. Man läßt das Reaktionsgemisch ca. 18 Stunden bei Raumtemperatur nachrühren und destilliert anschließend das Lösungsmittel sowie überschüssiges Amin im Vakuum ab.
Das erhaltene Rohprodukt kann direkt weiter zur Herstellung der Verbindung (IV-1) eingesetzt werden.

Nach Reinigung im Hochvakuum erhält man 7,0 g (84,5% der Theorie) L-Valin-4-methylpheneth-1-yl-amid. $^1H$-NMR ($D_6$-DMSO; $\delta$ = 0.74-0.87 (dm, 6H, 2x$CH_3$), 1.33 (d, 3H, $CH_3$), 1.62 (5, 2H, $NH_2$), 1.77-1.87 (m, H, CH), 2.26 (s, 3H, $CH_3$), 2.93 (d, H, CH), 4.85-4.95 (m, H, CH) 7.09-7.21 (m, 4H, Ph), 8.13 (m, H, NH).

Entsprechend Beispiel 1 und gemäß den allgemeinen Verfahrensangaben werden die folgenden Verbindungen der allgemeinen Formel (Ia) erhalten:

Tabelle 1

| Bsp.-Nr. | $R^1$ | Physikalische Daten |
|---|---|---|
| 2 | 4-Cl | Fp. 94-95°C |
| 3 | 4-$OCH_3$ | $n_D^{20}$ = 1.4905 |

**Herstellung der Verbindungen der Formel (IV)**

**Beispiel (IV-1)**

**(Verfahrensvariante a)**

1 g (0,0043 Mol) L-Valin-4-methylpheneth-1-yl-amid (vgl. Bsp. 1) und 0,62 g (0,0045 Mol) Kaliumcarbonat werden in 50 ml Dichlormethan bei -10°C suspendiert und mit 0,8 g (0,0065 Mol) Chlorameisensäureisopropylester in 10 ml Methylenchlorid versetzt. Man läßt das Reaktionsgemisch ca. 18 Stunden bei Raumtemperatur nachrühren, gießt auf Eiswasser, trennt die Phasen und extrahiert die wässrige Phase mehrmals mit Methylenchlorid. Die vereinigten organischen Phasen werden über Natriumsulfat getrocknet und eingeengt.

Man erhält 1,35 g (98% der Theorie) N-(Isopropoxy-carbonyl)-L-valin-4-methylpheneth-1-yl-amid vom Schmelzpunkt 160°C.

**(Verfahrensvariante b)**

In 300 ml Methylenchlorid werden 0,1 Mol L-Valin-4-methylpheneth-1-yl-amid (vgl. Bsp. 1) und 0,1 Mol Triethylamin bei -50°C vorgelegt. In diese Lösung werden 0,12 Mol Phosgen eingeleitet. Das Reaktionsgemisch wird innerhalb von 4 Stunden auf Raumtemperatur erwärmt. Anschließend wird eine Lösung aus 0,12 Mol Isopropanol in 50 ml Methylenchlorid hinzugetropft und 1 Stunde bei 50°C nachgerührt.

Nach Aufarbeitung erhält man 16,9 g N-(Isopropoxycarbonyl)-L-valin-4-methylpheneth-1-yl-amid vom Schmelzpunkt 160°C.

## Patentansprüche

**1.** Verbindungen der allgemeinen Formel

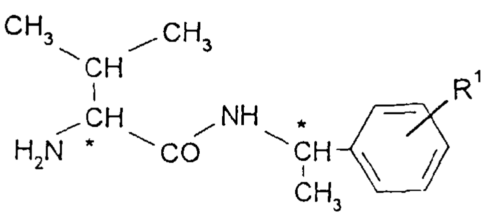

(I)

in welcher

$R^1$ für Chlor, Methyl, Ethyl oder Methoxy steht,

bzw. deren Salze.

**2.** Verfahren zur Herstellung von Verbindungen der Formel (I) wie in Anspruch 1 definiert bzw. deren Salze,
dadurch gekennzeichnet, daß man Oxazolidindione der Formel (II)

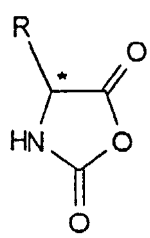

(II)

in welcher
R die oben angegebene Bedeutung hat
mit Aminen der allgemeinen Formel (III)

$$H_2N\text{-}A\text{-}X \qquad (III)$$

in welcher

A und X die in Anspruch 1 angegebenen Bedeutungen haben,

in Gegenwart eines Verdünnungsmittels und gegebenenfalls in Gegenwart eines Reaktionshilfsmittels umsetzt.

**3.** Schädlingsbekämpfungsmittel, gekennzeichnet durch einen Gehalt an mindestens einer Verbindung der Formel (I) nach Anspruch 1.

**4.** Verwendung von Verbindungen der Formel (I) nach Anspruch 1 zur Bekämpfung von Schädlingen.

**5.** Verfahren zur Bekämpfung von Schädlingen, dadurch gekennzeichnet, daß man Verbindungen der Formel (I) nach Anspruch 1 auf Schädlinge und/oder ihren Lebensraum einwirken läßt.

**6.** Verfahren zur Herstellung von Schädlingsbekämpfungsmitteln, dadurch gekennzeichnet, daß man Verbindungen der Formel (I) nach Anspruch 1 mit Streckmitteln und/oder oberflächenaktiven Mitteln vermischt.

**7.** Verfahren zur Herstellung von Aminosäure-Derivate der allgemeinen Formel (IV)

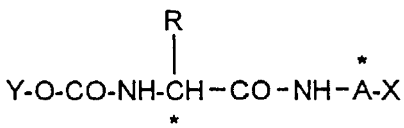

(IV)

in welcher

A für $CH(CH_3)$ steht

X für durch Chlor, Methyl, Ethyl oder Methoxy substituierter Phenylsteht, und

R für $CH(CH_3)_2$ steht,

Y für Aryl, Aralkyl oder Alkyl steht,

dadurch gekennzeichnet, daß man die Verbindungen der Formel (I),

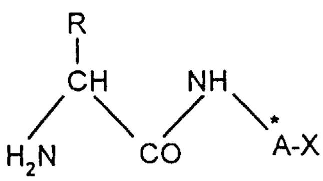

(I),

in welcher
R, A und X die oben angegebenen Bedeutungen haben,

a) mit Säure-Derivaten der allgemeinen Formel (V)

Y-O-CO-Z (V)

in welcher

Y die oben angegebene Bedeutung hat und

Z für Halogen oder die Gruppierung -O-CO-OY steht,

in Gegenwart eines Verdünnungsmittels sowie gegebenenfalls in Gegenwart eines Säurebindemittels und

gegebenenfalls in Gegenwart eines Katalysators bei Temperaturen zwischen -100 und +120°C umsetzt; oder

b) zunächst mit Phosgen in Gegenwart eines Verdünnungsmittels und gegebenenfalls in Gegenwart eines Säurebindemittels bei Temperaturen zwischen -100 und +120°C umsetzt undanschließend, ohne Isolierung der intermediär gebildeten Isocyanate bzw. Carbamoylchloride, mit Alkoholen der allgemeinen Formel (VI)

Y-OH (VI)

in welcher

Y die oben angegebene Bedeutung hat,

bei Temperaturen zwischen -100 und +120°C umsetzt.

**8.** Verfahren gemäß Anspruch 7, dadurch gekennzeichnet, daß man die Verbindungen der Formel (I) nach den Verfahren gemäß Anspruch 2 herstellt und die so erhaltenen Verbindungen der Formel (I), gegebenenfalls ohne Zwischenisolierung, direkt zu den Verbindungen der Formel (IV) umsetzt.

**9.** Verwendung von Verbindungen der Formel (I) wie in Anspruch 1 definiert als Zwischenprodukte zur Herstellung von Aminosäure-Derivaten der allgemeinen Formel (IV) mit in Anspruch 7 definiert.

## Claims

**1.** Compounds of the general formula

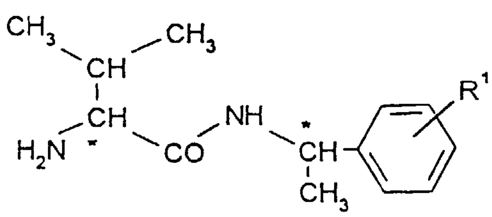

(I)

in which

$R^1$ represents chlorine, methyl, ethyl or methoxy, and their salts.

**2.** Process for preparing compounds of the formula (I) as defined in Claim 1 and their salts, characterized in that oxazolidinediones of the formula (II)

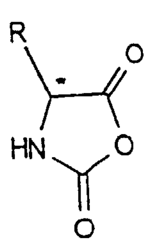

(II)

in which

R has the abovementioned meaning,

are reacted with amines of the general formula (III)

$H_2$-N-A-X (III)

in which

A and X have the meanings indicated in Claim 1.

in the presence of a diluent and, where appropriate, in the presence of a reaction aid.

**3.** Pesticide, characterized by a content of at least one compound of the formula (I) according to Claim 1.

**4.** Use of compounds of the formula (I) according to Claim 1 for controlling pests.

**5.** Method for controlling pests, characterized in that compounds of the formula (I) according to Claim 1 are allowed to act on pests or their habitat.

**6.** Process for preparing pesticides, characterized in that compounds of the formula (I) according to Claim 1 are mixed with extenders and/or surface-active agents.

**7.** Process for preparing amino acid derivatives of the general formula (IV)

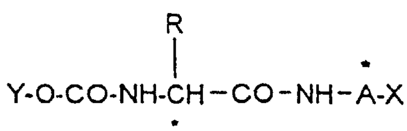

(IV)

in which

A represents $CH(CH_3)$

X represents phenyl substituted by chlorine, methyl, ethyl or methoxy, and

R represents $CH(CH_3)$

Y represents aryl, aralkyl or alkyl,

characterized in that the compounds of the formula (I),

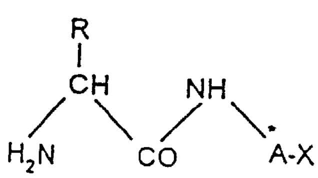

(I),

in which
R, A and X have the abovementioned meanings,

a) are reacted with acid derivatives of the general formula (V)

Y-O-CO-Z (V)

in which

Y has the abovementioned meaning, and

Z represents halogen or the group -O-CO-OY,

in the presence of a diluent and, where appropriate, in the presence of an acid-binding agent and, where appropriate, in the presence of a catalyst at temperatures between -100 and +120°C;
or

b) are reacted first with phosgene in the presence of a diluent and, where appropriate, in the presence of an acid-binding agent at temperatures between -100 and +120°C, and subsequently, without isolating the isocyanates or carbamoyl chlorides formed as intermediates, with alcohols of the general formula (VI)

Y-OH (VI)

in which

Y has the abovementioned meaning,

at temperatures between -100 and +120°C.

**8.** Process according to Claim 7, characterized in that the compounds of the formula (I) are prepared by the process according to Claim 2, and the compounds of the formula (I) obtained in this way are, where appropriate without intermediate isolation, reacted directly to compounds of the formula (IV).

**9.** Use of compounds of the formula (I) as defined in Claim 1 as intermediates for preparing amino acid derivatives of the general formula (IV) as defined in Claim 7.

**Revendications**

**1.** Composés de formule générale

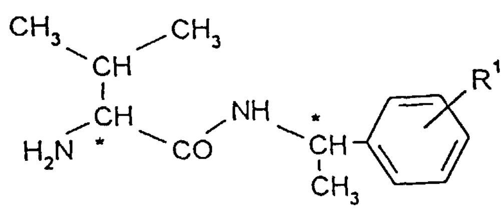

(I)

dans laquelle
R1 représente le chlore, un groupe méthyle, éthyle ou méthoxy,
ou leurs sels.

**2.** Procédé de production de composés de formule (I) suivant la revendication 1,
ou de leurs sels.
caractérisé en ce qu'on fait réagir des oxazolidindiones de formule (II)

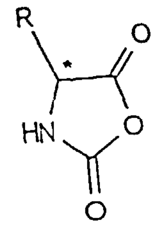

(II)

dans laquelle
R a la définition indiquée ci-dessus avec des amines de formule générale (III)

$$H_2N\text{-}A\text{-}X \qquad (III)$$

dans laquelle
A et X ont les définitions indiquées dans la revendication 1,
en présence d'un diluant et en la présence éventuelle d'une substance auxiliaire de réaction.

**3.** Compositions pesticides, caractérisées par une teneur en au moins un composé de formule (I) suivant la revendication 1.

**4.** Utilisation de composés de formule (I) suivant la revendication 1, pour combattre des parasites.

**5.** Procédé pour combattre des parasites, caractérisé en ce qu'on fait agir des composés de formule (I) suivant la revendication 1 sur les parasites et/ou sur leur milieu.

**6.** Procédé de préparation de compositions pesticides, caractérisé en ce qu'on mélange des composés de formule (I) suivant la revendication 1 avec des diluants et/ou des agents tensio-actifs.

**7.** Procédé de production de dérivés d'amino-acides de formule générale (IV)

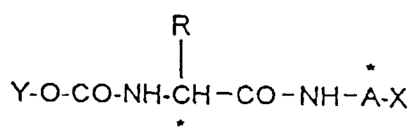

(IV)

dans laquelle

A représente un groupe $CH(CH_3)$
X représente un groupe phényle substitué par du chlore, un radical méthyle, éthyle ou méthoxy
R est un groupe $CH(CH_3)_2$
Y est un groupe aryle, aralkyle ou alkyle,

caractérisé en ce qu'on fait réagir des composés de formule (I)

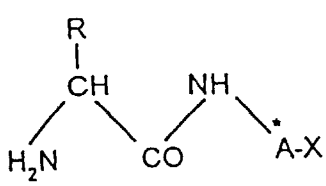

(I),

dans laquelle R, A, et X ont les définitions indiquées ci-dessus,

a) avec des dérivés d'acides de formule générale (V)

$$Y\text{-}O\text{-}CO\text{-}Z \qquad (V)$$

dans laquelle

Y a la définition indiquée ci-dessus et
Z représente un halogène ou le groupement -O-CO-OY

en présence d'un diluant de même qu'en la présence éventuelle d'un accepteur d'acide et le cas échéant, en présence d'un catalyseur, à des températures comprises entre -100 et +120°C ;
ou bien
b) tout d'abord avec le phosgène en présence d'un diluant et le cas échéant en présence d'un accepteur d'acide, à des températures comprises entre -100 et +120°C puis ensuite, sans isolement des isocyanates ou chlorures de carbamoyle formés au stade intermédiaire, avec des alcools de formule générale (VI)

$$Y\text{-}OH \qquad (VI)$$

dans laquelle

Y a la définition indiquée ci-dessus,

à des températures comprises entre -100 et +120°C.

**8.** Procédé suivant la revendication 7, caractérisé en ce qu'on prépare les composés de formule (I) par le procédé selon la revendication 2 et on transforme directement en les composés de formule (IV) les composés de formule (I) ainsi obtenus, le cas échéant sans isolement intermédiaire.

**9.** Utilisation de composés de formule (I) tels que définis dans la revendication 1 comme produits intermédiaires pour la préparation de dérivés d'amino-acides de formule générale (IV) telle que définie dans la revendication 7.